(19) Europäisches Patentamt
European Patent Office
Office européen des brevets

(11) Veröffentlichungsnummer: **0 340 702 B1**

(12) # EUROPÄISCHE PATENTSCHRIFT

(45) Veröffentlichungstag der Patentschrift: **07.01.93**

(51) Int. Cl.5: **C07C 47/565**, C07C 45/65

(21) Anmeldenummer: **89107872.7**

(22) Anmeldetag: **29.04.89**

(54) **Verfahren zur Herstellung von Hydroxybenzaldehyden.**

(30) Priorität: **05.05.88 DE 3815292**

(43) Veröffentlichungstag der Anmeldung:
**08.11.89 Patentblatt 89/45**

(45) Bekanntmachung des Hinweises auf die Patenterteilung:
**07.01.93 Patentblatt 93/01**

(84) Benannte Vertragsstaaten:
**BE CH DE ES FR GB IT LI NL**

(56) Entgegenhaltungen:
**DE-C- 2 904 315**

(73) Patentinhaber: **BASF Aktiengesellschaft**
**Carl-Bosch-Strasse 38**
**W-6700 Ludwigshafen(DE)**

(72) Erfinder: **Steck, Werner, Dr.**
**Auerstrasse 4**
**W-6700 Ludwigshafen(DE)**
Erfinder: **Lermer, Helmut, Dr.**
**D 3,4**
**W-6800 Mannheim 1(DE)**
Erfinder: **Rust, Harald**
**Dudostrasse 57**
**W-6730 Neustadt(DE)**
Erfinder: **Fritz, Gerhard, Dr.**
**Limburgstrasse 23**
**W-6701 Dannstadt-Schauernheim(DE)**
Erfinder: **Siegel, Hardo, Dr.**
**Hans-Purrmann-Allee 25**
**W-6720 Speyer(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Hydroxybenzaldehyden durch Spaltung von Phenolether-Brücken in aromatischen Aldehyden oder deren Acetalen in Gegenwart von zeolitischen Katalysatoren.

Für die Synthese von Hydroxybenzaldehyden, die wertvolle Zwischenprodukte für die Herstellung von Arzneimitteln wie Antibiotika, Agrarchemikalien, Glanzbildner für elektrochemische Metallisierungsbäder oder Farbstoffen darstellen oder als Geschmackstoffe bzw. Komponenten von künstlichen Riechstoffen Verwendung finden, sind bereits eine Reihe von Herstellmethoden beschrieben.

Die Spaltung von Phenolethern, die am Phenolring bereits eine Aldehydfunktion aufweisen, durch Säuren oder Säurengemische z. B. von HBr in Eisessig, Basen, mittels Pyridinium-chlorid, durch Lewis-Säuren wie $AlCl_3$ oder $BCl_3$ oder durch reduktive Methoden wie die Hydrogenolyse sind ausführlich im Houben-Weyl, Methoden der organischen Chemie (1976), Band 6/1, Seiten 314 bis 390 u. a., beschrieben.

Die Herstellung von p-Hydroxybenzaldehyd durch Behandlung von substituierten Benzaldehyd-dialkylacetalen mit wäßriger Halogenwasserstoffsäure ist Gegenstand der DE 29 04 315. Dort findet sich auch ein guter Überblick über den Stand der Herstellung des p-Hydroxybenzaldehydes nach verschiedenen Methoden (Spalte 1, Zeilen 35 bis 55). So führt u. a. Erhitzen von p-Methoxybenzaldehyd mit Pyridiniumchlorid auf 200 bis 220 °C zu p-Hydroxybenzaldehyd (Ber. dtsch. Chem. Ges. 74 (1974), 1219). Andererseits wird nachdrücklich hervorgehoben, daß die Behandlung p-Methoxybenzaldehyds mit HCl nicht zu p-Hydroxybenzaldehyd führt und auch eine HBr-Behandlung nur geringe Ausbeuten ermöglicht. Dies ist nicht verwunderlich, da Aldehyde in Gegenwart von Mineralsäuren zu Folgereaktionen neigen (Spalte 2, Zeilen 45 bis 55).

Eine weitere vielstufigen Synthese für p-Hydroxybenzaldehyd ist in DE 28 39 053 beschrieben, wobei p-Nitrotoluol zuerst in p-Aminotoluol überführt, dieses diazotiert und anschließend zum p-Hydroxybenzaldehyd hydrolysiert wird. Schließlich beschreibt J 6 2153 240 die Oxidation von p-Kresol zum p-Hydroxybenzaldehyd während in der J 6 2155 236 ein mehrstufiges Herstellverfahren durch Acetoxylierung, Halogenierung und Hydrolyse angegeben wird.

Eine Übersicht über die Herstellverfahren für Salicylaldehyd oder andere Hydroxybenzaldehyde findet sich bei Kirk-Othmer (Encyclopedia of Chemical Technology, Third Edition, Volume 13, S. 70-79). Viele der bekannten Herstellverfahren sind vielstufig und daher umständlich durchzuführen. Diese bekannten Verfahren werden alle homogenkatalytisch z.B. in Gegenwart von Mineralsäuren durchgeführt. Damit sind Nachteile wie die oft schwierige Abtrennung des Katalysators, bzw. dessen Verlust, und ausreichend kurze Kontaktzeiten nicht möglich. In vielen Fällen treten wegen des permanent vorhandenen oder nicht rasch genug abtrennbaren, Homogenkatalysators Folgereaktionen auf, welche das Wertprodukt zersetzen, oder die Ausbeuten stark verringern. So kann beispielsweise 4-Hydroxybenzaldehyd wegen seiner Instabilität gegenüber Säuren nicht aus Anisaldehyd-dialkylacetal durch homogene, saure Spaltung mit Salzsäure erhalten werden (siehe DE 29 04 315, Spalte 2, Zeilen 45 bis 55).

Es bestand daher die Aufgabe ein heterogen katalysiertes Herstellverfahren für Hydroxybenzaldehyde zu finden, das auf technisch einfachem Wege durchzuführen ist.

Es wurde nun gefunden, daß man bei der Herstellung von Hydroxybenzaldehyden der Formel (I) aus Benzaldehyden der Formel (II) oder deren Acetalen, in denen $R^1$ in der $R^1O$-Phenylethergruppe ein Alkyl-, Cycloalkyl-, Alkycycloalkyl- oder ein Alkylarylrest bedeutet und $R^2$ = H, OH oder mit $R^1$ identisch ist, die oben genannten Nachteile der bekannten Verfahren vermeidet, wenn man Benzaldehyde der Formel (II) oder deren Acetale, in denen $R^1$ und $R^2$ die vorgenannte Bedeutung aufweisen, in Anwesenheit von Wasser an Zeolithen als Heterogenkatalysatoren zu Hydroxybenzaldehyden der Formel (I) umsetzt.

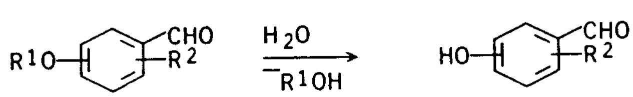


(II) (I)

Ganz besonders leicht lassen sich Monohydroxybenzaldehyde wie para-, meta- oder ortho-Hydroxybenzaldehyd aus den entsprechenden Alkoxybenzaldehyden oder deren Acetalen erhalten, wenn $R^1$ in der $R^1O$-Phenylethergruppe ein Alkylrest wie $-CH_3$ oder t.-Butyl bedeutet und $R^2$ = H ist.

Als Katalysatoren sind insbesondere die Zeolithe des Pentasil-Typs, z.B. die Aluminosilikatzeolithe, die Borosilikatzeolithe und die Eisensilikatzeolithe, aber auch Galliumsilikatzeolithe geeignet. Durch das erfindungsgemäße Verfahren kann man Phenolether, die am Phenylkern mindestens eine Aldehydfunktion aufweisen, heterogenkatalytisch an Zeolithen auf einfache Weise zu Hydroxybenzaldehyden spalten. Beispielsweise können so die technisch bedeutsamen 4- bzw. 2-Hydroxybenzaldehyde aus 4-Methoxybenzaldehyd und 2-Methoxybenzaldehyd bzw. deren Dialkylacetalen leicht erhalten werden. Die Nachteile der homogenkatalytischer. Vefahren, wie z.B. die aufwendige Katalysatorabtrennung oder die zumindest teilweise Zersetzung des Endproduktes durch den nicht rasch genug abtrennbaren Homogenkatalysator werden vermieden. Das erfindungsgemäße Verfahren ist kontinuierlich und mit kurzen Verweilzeiten und guten Ausbeuten durchführbar. Bei thermolabilen Ausgangsstoffen der Formel (II) ergibt sich dadurch eine Vermeidung oder Zurückdrängung der unerwünschten thermischen Decarbonylierung der Aldehydfunktion oder von Isomerisierungen, die als Nebenreaktionen ablaufen können.

Für das erfindungsgemäße Verfahren geeignete Ausgangsstoffe gemäß Formel (II) sind beispielsweise Monoalkoxybenzaldehyde wie 4-Methoxybenzaldehyd, 2-Methoxybenzaldehyd, 3-Methoxybenzaldehyd, 4-Ethoxybenzaldehyd, 4-tert.-Butyloxybenzaldehyd = [4-(1,1-Dimethylethoxy)benzaldehyd] und 4-Benzyloxybenzaldehyd = [4-(Phenylmethoxy)benzaldehyd] oder Monoalkoxyhydroxybenzaldehyde wie beispielsweise 3-Methoxy-4-Hydroxybenzaldehyd, 3-Hydroxy-4-Methoxy-benzaldehyd, 2-Hydroxy-3-Ethoxy-benzaldehyd.

Auch die Dialkylacetale, insbesondere die Dimethylacetale der genannten Aldehyde eignen sich gut.

Die für die erfindungsgemäße Umwandlung geeigneten Reaktionsbedingungen liegen abhängig vom Ausgangsstoff bei der Gasphasenreaktion bei 120 bis 370 ° C und einer Belastung WHSV von 0,1 bis 15 $h^{-1}$ (WHSV = g Ausgangsgemisch/g Katalysator und Stunde). Die Reaktion kann bei Normaldruck im Festbett, Fließ- oder Wirbelbett ausgeführt werden.

Dabei empfiehlt es sich je nach Reaktionstemperatur und Thermostabilität der Stoffe die Kontaktzeit der Reaktionspartner am Katalysator zu verkürzen und/oder das Reaktionsgemisch nach dem Durchlaufen der Katalysatorschüttung schnell abzukühlen. Auf diese Weise kann man auch bei thermolabilen Ausgangs- und/oder Endprodukten ausbeutemindernde Nebenreaktionen, beispielsweise Decarbonylierungen vermeiden oder zumindest stark zurückdrängen.

Auch unter vermindertem Druck z.B. mittels einer dem Katalysatorbett austragseitig nachgeschalteten Vakuumpumpe, können aufgrund der dadurch kurzen, auch im Bruchteil von Sekunden liegenden Verweilzeiten am Katalysator die Selektivität und die Ausbeuten vorteilhaft beeinflußt werden.

Es ist auch möglich, die Reaktion in der Flüssigphase bei Temperaturen zwischen 120 und 300 ° C und einer WHSV von 0.1 bis 15 $h^{-1}$ durchzuführen. Je nach Flüchtigkeit der Ausgangsverbindungen kann die Umsetzung bei Normaldruck oder bei Drücken von Normaldruck bis 40 bar durchgeführt werden.

Die Durchführung erfolgt vorzugsweise kontinuierlich, wobei auch eine Kreisgasfahrweise mit vollständiger oder teilweiser Rückführung des Reaktionsaustrages angebracht sein kann.

Schwerflüchtige, flüssige oder feste Ausgangsstoffe werden vorteilhaft in gelöster oder emulgierter Form, z.B. in Alkoholen, Tetrahydrofuran, Toluol- oder Petrolether, eingesetzt. Allgemein ist eine Verdünnung aller Ausgangsstoffe, auch der leichterflüchtigen Lösungsmitteln oder in der Gasphase mit Inertgasen, wie $N_2$, Ar, He oder $H_2O$-Dampf möglich. Bei einem flüssigen Ausgangsstoff der Formel (II), z.B. bei 4-Methoxybenzaldehyd, kann nach Wasserzusatz bereits oft durch Rühren eine feinverteilte Emulsion des Wassers im Methoxybenzaldehyd erhalten werden. Diese Emulsion kann dann mittels geeigneter Dosierpumpen direkt in der gewünschten Menge in den Reaktor eingeführt und dort umgesetzt werden.

Der Austrag an Endprodukten ist bei Verwendung von Wasser häufig zweiphasig, so daß sich überschüssiges oder durch Nebenreaktionen gebildetes Wasser leicht von der organischen Phase abtrennen läßt. Bei Gegenwart von Alkoholen, wie Methanol, oder von Gemischen aus Ausgangsstoff, Wasser und Lösungsmittel werden entweder homogene Gemische oder wie vorstehend beschrieben pump- und dosierfähige Emulsionen erhalten.

Bei Verwendung der Dialkylacetale der Ausgangsstoffe (II), beispielsweise von 2- oder 4-Methoxybenzaldehyddimethylacetalen, können diese selbst oder im Lösungsmittel gelöst in Anwesenheit von Wasser über das Zeolith-Katalysatorbett geleitet werden.

In anderen Fällen ist es vorteilhaft, zweistufig zu arbeiten, indem zuerst an einem sauren, heterogenen Katalysator die Diacetale der Alkoxybenzaldehyde in Gegenwart von Wasser vollständig oder teilweise in die entsprechenden Alkoxybenzaldehyde umgewandelt werden und anschließend im gleichen oder in einem weiteren Reaktor der Aldehyd-haltige Austrag, weiter zu Hydroxybenzaldehyden umgesetzt wird.

Nach der Umsetzung werden die Produkte aus dem Austrag durch übliche Verfahren, z.B. durch Destillation, Extraktion oder Ausfrieren aus dem Reaktionsgemisch isoliert; nichtumgesetzte Ausgangsstoffe werden gegebenenfalls in die Umsetzung zurückgeführt. Wenn die Hydroxybenzaldehyde ausreichend saure Hydroxygruppen aufweisen, beispielsweise 4-Hydroxybenzaldehyd, dann können diese leicht durch ein Zweiphasengemisch von Toluol und wäßriger Natronlauge getrennt werden, da sich der Methoxybenzaldehyd im Toluol, der Hydroxybenzaldehyd dagegen in der Natronlauge anreichern. Nach Neutralisation der

wäßrigen, alkalischen Fraktion und Entfernung des Wassers kann der Hydroxybenzaldehyd mit einem Lösungsmittel wie Methanol leicht vom festen Rückstand abgetrennt werden.

Als Katalysatoren für das erfindungsgemäße Verfahren werden Zeolithe eingesetzt. Zeolithe sind kristalline Aluminosilikate, die eine hochgeordnete Struktur mit einem dreidimensionalen Netzwerk von $SiO_4$- oder $AlO_4$-Tetraedern besitzen, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere Elemente wie B, Ga, Fe, Cr, V, As, Sb oder Be in das Gitter eingebaut werden, oder das Silicium kann durch ein vierwertiges Element wie Ge, Ti, Zr, Hf ersetzt werden.

Entsprechend ihrer Struktur werden die Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man Zeolithe vom Typ A, L, X oder Y.

Für das erfindungsgemäße Verfahren besonders geeignet sind Zeolithe vom Pentasiltyp. Diese haben als Grundbaustein einen aus $SiO_4$-Tetraedern aufgebauten Fünfring. Sie sind durch ein hohes $SiO_2/Al_2O_3$-Verhältnis - auch Modul genannt - gekennzeichnet, sowie durch Porengrößen, die zwischen denen der Zeolithe vom Typ A und denen vom Typ X oder Y liegen. Für das erfindungsgemäße Verfahren haben sich beispielsweise Pentasil-Zeolithe mit $SiO_2/Al_2O_3$-Verhältnissen zwischen 15 und 700 als gut geeignet erwiesen. Die Herstellung dieser Pentasil Zeilithe wird u.a. in der US-PS 3 702 886 beschrieben. Die Herstellung der analogen Borosilkatzeolithe vom Typ AMS-1B ist Gegenstand der DE 27 46 790.

Die erfindungsgemäß verwendbaren Zeolithe können unterschiedliche chemische Zusammensetzung aufweisen, geeignet sind z.B. Alumino-, Boro-, Eisen-, Beryllium-, Gallium-, Chrom-, Arsen-, Antimon- und Wismutsilikatzeolithe oder deren Gemische sowie Alumino-, Boro-, Gallium-, Eisengermanatzeolithe oder deren Gemische. Insbesondere eignen sich die Alumino-, Boro- oder Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Diaminohexan oder 1,3-Diaminopropan oder Triethylentetramin-Lösung mit oder insbesondere ohne Alkali- oder Erdalkalizusatz bei 100 bis 220 °C unter autogenem Druck hergestellt. Geeignet sind auch isotaktische Zeolithe nach EP 0 034 727. Die erhaltenen Aluminosilikatzeolithe enthalten je nach Wahl der Ausgangsstoffmengen ein $SiO_2/Al_2O_3$-Verhältnis von 10 bis 40 000.

Diese Aluminosilikatzeolithe kann man in etherischem Medium wie Diethylenglykoldimethylether, in alkoholischem Medium wie Methanol oder 1,4-Butandiol oder in Wasser synthetisieren.

Der Borosilikatzeolith wird z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Diaminohexan oder 1,3-Diaminopropan oder Triethylentetramin-Lösung mit und insbesondere ohne Alkali- oder Erdalkalizusatz zur Reaktion bringt. Auch isotaktische Borosilikatzeolithe, nach EP 0 034 727 kann man verwenden. Man kann auch Borosilikatzeolithe verwenden, die statt aus wäßriger Aminlösung aus einer etherischen Lösung, z.B. Diethylenglykoldimethylether oder aus alkoholischer Lösung, z.B. 1,6-Hexandiol auskristallisiert werden. Die Herstellung von Borzeolith ist z.B. im EP 0 007 081 beschrieben.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Diaminohexan mit oder ohne Alkali- oder Erdalkalizusatz bei 100 bis 200 °C unter autogenem Druck. Die Herstellung ist beschrieben in DE 28 31 611.

Zu den verwendbaren siliciumreichen Zeolithen ($SiO_2/Al_2O_3$ größer oder gleich 10) gehören auch die bekannten ZSM-Typen sowie Ferrierit (EP 0 012 473) und NU-1 (US PS 4 060 590) sowie Silicalite® (US-PS 4 061 724).

Die so hergestellten Alumino-, Boro- und Eisensilikatzeolithe können nach ihrer Isolierung, Trocknung bei 100 bis 160 °C und Calcinierung bei 450 bis 550 °C, mit einem Bindemittel im Massenverhältnis Zeolith zu Bindemittel 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, $SiO_2$, $TiO_2$, Gemische aus hochdispersem $SiO_2$ und hochdispersem $Al_2O_3$ sowie Ton. Nach der Verformung werden die Extrudate oder Preßlinge bei 110 °C/16 h getrocknet und bei

500 ° C/16 h calciniert.

Man erhält auch geeignete Katalysatoren, wenn der isolierte Alumino- bzw. Borosilikatzeolith direkt nach der Trocknung verformt wird und erst nach der Verformung einer Calcinierung unterworfen wird. Die Alumino- und Borosilikatzeolithe können in reiner Form, ohne Binder, als Stränge oder Tabletten eingesetzt werden, wobei als Verstrangungs- oder Peptisierungsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen, und anschließende Calcinierung oder durch Behandlung mit Säuren vollkommen oder partiell in die gewünschte H-Form überführt werden.

Wenn bei der Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 ° C, bevorzugt 500 ° C, zu regnerieren. Die Zeolithe erhalten dadurch in der Regel ihre Anfangsaktivität zurück.

Durch gezielte, partielle Verkokung (pre-coke) ist es auch möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren, wobei der Gehalt an den Modifizierungselementen so zu bemessen ist, daß eine ausreichende Aktivität der Katalysatoren gewährleistet ist.

Eine geeignete Modifizierung der Katalysatoren besteht z.B. darin, daß man den unverformten oder verformten Zeolithen mit Metallsalzen durch Ionenaustausch oder durch Imprägnierung dotiert. Als Metalle werden Alkalimetalle, wie Li, K, Cs, Erdalkalimetalle, wie Mg, Ca, Ba; Erdmetalle, wie B, Al, Ga; Übergangsmetalle, wie Cu, Zn, Cr, Mn, Fe, Co, Ni, W und Mo und seltene Erdmetalle wie Ce, La, Pr, Nd, eingesetzt.

Auch eine Modifizierung mit Phosphor durch Behandlung mit Phosphorsäuren, Phosphorsäureestern wie Trimethylphosphat ist möglich.

Zweckmäßigerweise führt man die Dotierung so durch, daß man den verformten Zeolithen in einem Steigrohr vorlegt und bei 20 bis 100 ° C, z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium-, Alkaliform des Zeolithen vorgenommen werden. Eine weitere Möglichkeit der Metallaufbringung auf den Zeolithen ist gegeben, indem man das zeolithische Material mit einem Halogenid, einem Nitrat, einem Carbonat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Magnesiumacetat in Wasser löst. Mit dieser Lösung wird der verformte oder unverformte Zeolith etwa 30 Minuten getränkt. Die überstehende Lösung wird am Rotationsverdampfer von Wasser befreit. Danach wird der getränkte Zeolith bei etwa 130 ° C getrocknet und bei 500 ° C calciniert. Dieser Tränkvorgang kann mehrmals hintereinander vorgenommen werden, um den gewünschten Metallgehalt einzustellen.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine wäßrige Lösung von Ammoniumchlorid bei leicht erhöhter Temperatur zwischen 30 und 80 ° C im Kreislauf 15 bis 20 h leitet. Danach wird mit Wasser ausgewaschen, bei etwa 110 ° C getrocknet und bei etwa 500 ° C calciniert.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 h bei 80 ° C behandelt. Nach der Behandlung wird mit Wasser gewaschen, bei 100 ° C/16 h getrocknet und bei 500 ° C/20 h calciniert.

Nach einer anderen Arbeitsweise behandelt man Zeolithe vor oder nach ihrer Verformung mit Bindemitteln, z.B. 1 bis 3 h bei Temperaturen von 60 bis 80 ° C mit einer 3- bis 25 Gew.%igen, insbesondere 12- bis 20 Gew.%igen wäßrigen Salzsäure. Anschließend wird der so behandelte Zeolith mit Wasser gewaschen, getrocknet und bei 400 bis 500 ° C calciniert.

Eine besondere Ausführungsform für die Säurebehandlung besteht darin, daß man das zeolithische Material vor seiner Verformung bei erhöhter Temperatur mit 0,001 n bis 2 n, vorzugsweise 0,05 n bis 0,5 n Flußsäure behandelt, beispielsweise durch Erhitzen unter Rückfluß über einen Zeitraum von 0,5 bis 5 h, vorzugsweise 1 bis 3 h.

Nach Isolierung, z.B. durch Abfiltrieren und Auswaschen, des zeolithischen Materials wird dieses

zweckmäßig, bei Temperaturen von 100 °C bis 160 °C, getrocknet und bei Temperaturen von 450 °C bis 600 °C calciniert.

Gemäß einer weiteren bevorzugten Ausführungsform für die Säurebehandlung wird das zeolithische Material nach seiner Verformung mit Bindemittel bei Temperaturen von 50 °C bis 90 °C, 1 bis 3 h mit 3 - 25 Gew.%iger, vorzugsweise mit 12 - 20 Gew.%iger Salzsäure, behandelt. Anschließend wird das zeolithische Material ausgewaschen und bei 100 bis 160 °C, getrocknet und bei 450 °C bis 600 °C calciniert. Auch eine aufeinanderfolgende Behandlung mit HF und HCl kann vorteilhaft sein.

Nach einer anderen Arbeitsweise kann man Zeolithe durch Aufbringen von Phosphorverbindungen, wie Trimethylphosphat, primären, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut eingesetzt werden.

Versuche in der Gasphase

Die Reaktion in der Gasphase wird unter isothermen Bedingungen in einem Rohrreaktor (Wendel, 0,6 cm Innendurchmesser, 90 cm Länge) mehrere Stunden lang an einem Katalysatorfestbett durchgeführt. Dabei wird die Katalysatormenge zwischen 1 und 10 g, entsprechend einer Belastung WHSV zwischen von 10 und 0.5/h variiert. Die Reaktionsprodukte werden durch übliche Methoden abgetrennt und durch GC/MS, NMR, den Schmelz- oder Siedepunkt charakterisiert. Die quantitative Bestimmung der Reaktionsprodukte und der Ausgangsstoffe erfolgt gaschromatographisch oder durch Auswiegen der durch Destillation oder Extraktion erhaltenen Fraktionen.

Versuche in der Flüssigphase

In den vorstehend beschriebenen Rohrreaktor werden an geeigneten Stellen Druckhalte- und Sicherheitsventile eingebaut. Die als Lösung oder Emulsion vorliegenden Einsatzstoffe werden in diesem Fall mit einer handelsüblichen Hochdruckpumpe zudosiert.

Herstellung der Katalysatoren

Katalysator 1:

Ein Eisensilicatzeolith vom Pentasil-Typ wird unter hydrothermalen Bedingungen bei autogenem Druck und 165 °C aus 273 g Natrium-Wasserglas (27,2 % $SiO_2$, 8,5 % $Na_2O$), 126 g 1,6-Diaminohexan, 551 g Wasser, 20,6 g 96%iger Schwefelsäure und 31,1 g Eisen(III)-sulfat in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Eisensilicatzeolith setzt sich aus 84,5 Gew.% $SiO_2$, 12,1 Gew.% $Fe_2O_3$ und 0.074 Gew.% Na zusammen. Mit diesem Material werden durch Verformen mit einem Verformungshilfsmittel 4-mm-Stränge hergestellt, die bei 110 °C/16 h getrocknet und bei 500 °C/24 h calciniert werden.

Die Stränge werden viermal mit 20 Gew.%iger $NH_4Cl$-Lösung (15 ml Lösung/g Formkörper) bei 80 °C in einem Steigrohr ausgetauscht. Anschließend wird chloridfrei gewaschen. Die Stränge werden bei 110 °C 10 h getrocknet und anschließend bei 500 °C 5 h calciniert. Der Na-Gehalt beträgt nun 0.015 Gew.%.

Katalysator 2:

Ein Aluminosilicatzeolith vom ZSM-5-Typ wird unter hydrothermalen Bedingungen bei autogenem Druck und 175 °C aus 1,0 kg pyrogener Kieselsäure, 48,0 g Natriumaluminat (58,6 Gew.% $Al_2O_3$, 40,0 Gew.% $Na_2O$), 44,0 g Natriumhydroxid, 1,69 kg wäßriger Tetra-n-propylammoniumhydroxid-Lösung (20 %ig) und 16,6 kg Wasser in einem Rührautoklaven hergestellt. Nach Abfiltrieren und Auswaschen wird das kristalline Reaktionsprodukt bei 110 °C/24 h getrocknet und bei 500 °C 24 h calciniert. Dieser Zeolith ist durch folgende Zusammensetzung charakterisiert: 85,9 Gew.% $SiO_2$, 2,3 Gew.% $Al_2O_3$, 0,32 Gew.% Na.

Mit diesem Zeolith werden durch Verformen mit einem Verformungshilfsmittel 2-mm-Stränge hergestellt. Diese werden bei 110 °C 16 h getrocknet und bei 500 °C 16 h calciniert.

Die Stränge werden viermal mit 20 Gew.%iger $NH_4Cl$-Lösung (15 ml Lösung/g Formkörper) bei 80 °C

in einem Steigrohr ausgetauscht. Anschließend wird chloridfrei gewaschen. Die Stränge werden bei 110 °C 10 h getrocknet und anschließend bei 500 °C 5 h calciniert. Der Na-Gehalt beträgt nun 0,007 Gew.%.

Katalysator 3:

Ein Borosilikatzeolith vom ZSM-5-Typ wird unter hydrothermalen Bedingungen bei autogenem Druck und 165 °C aus 6,0 kg Kieselsol (30 Gew.% $SiO_2$), 118 g Borsäure, 252 g Natriumhydroxid, 1,44 kg Tetra-n-propylammoniumbromid und 14,7 kg Wasser in einem Rührautoklaven hergestellt. Nach Abfiltrieren und Auswaschen wird das kristalline Reaktionsprodukt bei 110 °C/24 h getrocknet und bei 500 °C 24 h calciniert. Dieser Zeolith ist durch folgende Zusammensetzung charakterisiert: 86,3 Gew.% $SiO_2$, 1,38 Gew.% $B_2O_3$, 0,65 Gew.% Na.

Mit diesem Zeolith werden durch Verformen mit einem Verformungshilfsmittel 2-mm-Stränge hergestellt. Diese werden bei 110 °C 16 h getrocknet und bei 500 °C 16 h calciniert.

Die Stränge werden viermal mit 20 Gew.%iger $NH_4Cl$-Lösung (15 ml Lösung/g Formkörper) bei 80 °C in einem Steigrohr ausgetauscht. Anschließend wird chloridfrei gewaschen. Die Stränge werden bei 110 °C 10 h getrocknet und anschließend bei 500 °C 5 h calciniert. Der Na-Gehalt beträgt nun 0,015 Gew.%.

Katalysator 4:

Ein Aluminosilicatzeolith vom ZSM-5-Typ wird unter hydrothermalen Bedingungen bei autogenem Druck und 170 °C aus 13,2 kg Kieselsol, welches aufgrund seiner Herkunft Spuren von Aluminium enthält, (30 Gew.% $SiO_2$), 314 g Natriumhydroxid, 3,36 kg wäßriger Tetra-n-propylammoniumhydroxid-Lösung (20 %ig) und 6,4 kg Wasser in einem Rührautoklaven hergestellt. Nach Abfiltrieren und Auswaschen wird das kristalline Reaktionsprodukt bei 110 °C/24 h getrocknet und bei 500 °C 24 h calciniert. Dieser Zeolith ist durch folgende Zusammensetzung charakterisiert: 81,4 Gew.% $SiO_2$, 0,20 Gew.% $Al_2O_3$, 1,0 Gew.% Na.

Mit diesem Zeolith werden durch Verformen mit einem Verformungshilfsmittel 2-mm-Stränge hergestellt. Diese werden bei 110 °C 16 h getrocknet und bei 500 °C 16 h calciniert.

Die Stränge werden viermal mit 20 Gew.%iger $NH_4Cl$-Lösung (15 ml Lösung/g Formkörper) bei 80 °C in einem Steigrohr ausgetauscht. Anschließend wird chloridfrei gewaschen. Die Stränge werden bei 110 °C 10 h getrocknet und anschließend bei 500 °C 5 h calciniert. Der Na-Gehalt beträgt nun 0,02 Gew.%.

Katalysator 5:

Aus dem als Katalysator 4 erhaltenen Aluminosilikatzeolith werden durch Verformen mit pyrogener Kieselsäure (8 Gewichtsteile : 2 Gewichtsteile) und einem Verformungshilfsmittel 2-mm-Stränge hergestellt, die bei 110 °C/16 h getrocknet und bei 500 °C 16 h calciniert werden.

Die Stränge werden viermal mit 20 Gew.%iger $NH_4Cl$-Lösung (15 ml Lösung/g Formkörper) bei 80 °C in einem Steigrohr ausgetauscht. Anschließend wird chloridfrei gewaschen. Die Stränge werden bei 110 °C 10 h getrocknet und anschließend bei 500 °C 5 h calciniert. Der Na-Gehalt beträgt nun 0,018 Gew.%.

Katalysator 6:

Der Borosilkatzeolith des Pentasil-Typs wird in einer hydrothermalen Synthese aus 650 g hochdispersem $SiO_2$, 373 g $H_3BO_3$, 8 000 g einer wäßrigen 1,6-Diaminohexan-Lösung (50 Gew.% Amin) bei 165 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 95,4 Gew.% $SiO_2$ und 3,31 Gew.% $B_2O_3$.

Mit diesem Material werden durch Verformen mit einem Verformungshilfsmittel 2-mm-Stränge hergestellt, die bei 110 °C/16 h getrocknet und bei 500 °C 16 h calciniert werden.

Katalysator 7:

Ein Aluminosilikatzeolith vom Pentasil-Typ wird unter hydrothermalen Bedingungen bei autogenem Druck und 150 °C aus 650 g hochdispersem $SiO_2$, 203 g $Al_2(SO_4)_3$ x 18 $h_2O$ in 11,3 kg einer wäßrigen 1,6-Diaminohexan-Lösung (42 Gew.% Amin) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 92,6 Gew.% $SiO_2$ und 4,6 Gew.% $Al_2O_3$.

Mit diesem Material werden durch Verformen mit einem Verformungshilfsmittel 2-mm-Stränge hergestellt, die bei 110 ° C 16 h getrocknet und bei 500 ° C 16 h calciniert werden.

Katalysator 8:

Katalysator 8 ist ein handelsübliches $Al_2O_3$ (D 10-10®).

Katalysator 9:

Katalysator 9 ist ein handelsübliches $SiO_2$ (D 11-10®).

Katalysator 10:

Aus dem bei Katalysator 3 erhaltenen Borosilikatzeolithen werden durch Verformen mit pyrogener Kieselsäure (8 Gewichtsteile : 2 Gewichtsteile) und einem Verformungshilfsmittel 2-mm-Stränge hergestellt, die bei 110 ° C/16 h getrocknet und bei 500 ° C/16 h calciniert werden.

Die Stränge werden viermal mit 20 Gew.%iger $NH_4Cl$-Lösung (15 ml Lösung/g Formkörper) bei 80 ° C in einem Steigrohr ausgetauscht. Anschließend wird chloridfrei gewaschen. Die Stränge werden bei 110 ° C 10 h getrocknet und anschließend bei 500 ° C 5 h calciniert. Der Na-Gehalt beträgt nun 0,02 Gew.%.

Katalysator 11:

Der Borosilikatzeolith des Pentasil-Typs wird in einer hydrothermalen Synthese aus 740 g hochdispersem $SiO_2$, 360 g $H_3BO_3$, 9,7 kg einer wäßrigen 1,6-Diaminohexan-Lösung (50 Gew.% Amin) bei 165 ° C unter autogenem Druck in einem Rührautoklaven hergestellt. Nach Abfiltrieren und Auswaschen wird das kristalline Reaktionsprodukt bei 160 ° C 24 h getrocknet und bei 500 ° C 24 h calciniert. Dieser Borosilikatzeolith setzt sich zusammen aus 92,9 Gew.% $SiO_2$ und 2,92 Gew.% $B_2O_3$.

Der Borosilikatzeolith wird mit 0,08 n HF (3,6 g HF-Lösung/g Zeolith) 1 h unter Rückfluß gekocht. Danach wird der Zeolith abfiltriert, gewaschen, bei 110 ° C getrocknet und bei 500 ° C 5 h calciniert.

Der behandelte Borosilikatzeolith wird mit einem amorphen Aluminosilikat (Massenverhältnis $SiO_2$ : $Al_2O_3$ = 75 : 25) im Massenverhältnis 60 : 40 unter Zusatz eines Verformungshilfsmittels zu 2-mm-Stränge verformt, die bei 110 ° C/16 h getrocknet und bei 500 ° C 16 h calciniert werden.

Der F-Gehalt des Katalysators beträgt 0,055 Gew.%.

Katalysator 12:

Ein Aluminosilikatzeolith vom Pentasil-Typ wird unter hydrothermalen Bedingungen bei autogenem Druck und 150 ° C aus 650 g hochdispersem $SiO_2$, 203 g $Al_2(SO_4)_3$ x 18 $H_2O$ in 11,9 kg einer wäßrigen 1,6-Diaminohexan-Lösung (46 Gew.% Amin) in einem Rührautoklaven hergestellt. Nach Abfiltrieren und Auswaschen wird das kristalline Reaktionsprodukt bei 110 ° C 24 h getrocknet und bei 500 ° C 24 h calciniert. Dieser Aluminosilikatzeolith enthält 89,5 Gew.% $SiO_2$ und 2,7 Gew.% $Al_2O_3$.

Mit diesem Material werden durch Verformen mit einem Verformungshilfsmittel 2-mm-Stränge hergestellt, die bei 110 ° C 16 h getrocknet und bei 500 ° C 16 h calciniert werden.

Die Stränge werden mit einer wäßrigen $Mg(OOC\text{-}CH_3)_2$ . 4 $H_2O$-Lösung getränkt, bei 130 ° C 2 h getrocknet und bei 540 ° C 2 h calciniert. Der Mg-Gehalt beträgt 2,0 Gew.%.

Katalysator 13:

Man verformt den Borosilikatzeolith des Katalysators 11 mit einem Verformungshilfsmittel zu 2-mm-Stränge, die bei 110 ° C getrocknet und bei 500 ° C 16 h calciniert werden.

Die Stränge werden mit Trimethylphosphat, gelöst in Wasser, 30 min. getränkt. Der P-Gehalt beträgt nach Trocknung bei 110 ° C 2 h 1,73 Gew.%.

Katalysator 14:

Der Borosilikatzeolith des Pentasil-Typs wird in einer hydrothermalen Synthese aus 552 g hochdispersem $SiO_2$, 331 g $H_3BO_3$, 10,5 kg einer wäßrigen Triethylentetramin-Lösung (47 Gew.% Amin) bei 175 ° C unter autogenem Druck in einem Rührautoklaven hergestellt. Nach Abfiltrieren und Auswaschen wird das kristalline Reaktionsprodukt bei 140 ° C/24 h getrocknet und bei 500 ° C/24 h calciniert. Dieser Borosilikatzeolith setzt sich zusammen aus 88,0 Gew.% $SiO_2$ und 5,23 Gew.% $B_2O_3$.

Mit diesem Material werden durch Verformen mit einem Verformungshilfsmittel 2-mm-Stränge hergestellt, die bei 110 ° C/16 h getrocknet und bei 500 ° C 16 h calciniert werden.

Katalysator 15:

Aus dem Borosilikatzeolith des Katalysators 11 werden durch Verformen mit einem Verformungshilfsmittel 2-mm-Stränge hergestellt, die bei 110 ° C/16 h getrocknet und bei 500 ° C 16 h calciniert werden.

Die Stränge werden mit einer wäßrigen $Mg(OOC\text{-}CH_3)_2 \cdot 4\ H_2O$-Lösung getränkt, wie bei Katalysator 12. Der Mg-Gehalt beträgt 1,1 Gew.%.

Katalysator 16:

Die bei Katalysator 6 hergestellten Borosilikatzeolith-Stränge werden mit $La(NO_3)_3 \cdot 6\ H_2O$-Lösung, wie bei Katalysator 12, getränkt (La = 1,8 Gew.%).

Katalysator 17:

Der als Katalysator 11 hergestellte Borosilikatzeolith wird mit Pseudoboehmit im Gewichtsverhältnis 60 : 40 verstrangt, bei 110 ° C/16 h getrocknet und bei 500 ° C/16 h calciniert. Die Stränge werden mit $Cr(NO_3)_3 \cdot 9\ H_2O$-Lösung, wie bei Katalysator 12, getränkt (Cr = 1,90 Gew.%).

Beispiele 1 bis 20

Gasphasenreaktionen zur Herstellung von 4-Hydroxybenzaldehyd (I) aus 4-Methoxybenzaldehyd (II)

Ausgangsstoff A:

Gemisch aus 4-Methoxybenzaldehyd (Anisaldehyd), Methanol und Wasser im Molverhältnis 1 : 1.5 : 1.5

Dem vorstehend beschriebenen Rohrreaktor werden bei Normaldruck mit einer Dosierpumpe 10 ml/h des Ausgangsstoffes A unter gleichzeitigem Inertisieren mit einem Stickstoffstrom kontinuierlich zugeführt. Das Katalysatorbett wird dabei von unten nach oben von den gasförmig vorliegenden Edukten und Reaktionsprodukten aufsteigend durchströmt. Nach der Umsetzung in der Gasphase am Katalysator - die gewählte Belastung WHSV in $h^{-1}$, die Reaktionstemperatur im Katalysatorbett in [ ° C] und der verwendete Katalysator sind in der Tabelle 1 aufgeführt - wird der Reaktionsaustrag in einer Glasapparatur kondensiert und nach der in Stunden [h] angegebenen Laufzeit gaschromatisch analysiert ((I) = 4-Hydroxybenzaldehyd: (II) = 4-Methoxybenzaldehyd; Nebenprodukte Anisol ( = III) und Phenol ( = IV). Die Umsätze und Selektivitäten sind in Flächenprozenten angegeben.

Tabelle 1

| Beispiel | Katalysator | WHSV/h | $N_2$ [Nl/h] | Laufzeit [h] | Temp. [°C] | GC-Analyse des Austrags | | | | |
|---|---|---|---|---|---|---|---|---|---|---|
| | | | | | | Umsatz an (II) [%] | Menge an (I) [Fl.-%] | Selekt. in [%] | | |
| | | | | | | | | (I) | (III) | (IV) |
| 001 | 1 | 1.4 | 5.0 | 8 | 270 | 46.0 | 17.8 | 38.6 | 36.4 | 25.1 |
| | 1 | 1.4 | 5.0 | 28 | 270 | 28.2 | 15.5 | 55.1 | 27.7 | 17.3 |
| 002 | 2 | 1.4 | 5.0 | 6 | 270 | 20.4 | 10.0 | 53.4 | 34.4 | 10.2 |
| 003 | 3 | 1.4 | 5.0 | 7 | 270 | 37.1 | 17.3 | 46.5 | 33.4 | 20.1 |
| | 3 | 1.4 | 5.0 | 31 | 270 | 24.0 | 15.4 | 64.3 | 24.3 | 11.3 |
| 004 | 4 | 1.4 | 5.0 | 2 | 210 | 4.1 | 3.5 | 84.8 | 7.1 | 0.0 |
| 005 | 4 | 1.4 | 5.0 | 2 | 240 | 32.2 | 16.4 | 51.5 | 30.5 | 18.0 |
| 006 | 4 | 1.4 | 5.0 | 8 | 270 | 31.3 | 16.4 | 52.6 | 30.5 | 16.9 |
| 007 | 4 | 1.4 | 5.0 | 28 | 270 | 22.1 | 13.9 | 62.7 | 23.0 | 11.8 |
| 008 | 4 | 1.4 | 5.0 | 14 | 350 | 50.2 | 5.7 | 11.3 | 54.5 | 25.8 |
| 009 | 4 | 1.4 | 50.0 | 4 | 270 | 29.4 | 11.7 | 39.9 | 38.3 | 21.7 |
| 010 | 4 | 2.8 | 50.0 | 4 | 270 | 21.5 | 5.2 | 24.1 | 42.3 | 33.5 |
| 011 | 4 | 10.0 | 50.0 | 4 | 270 | 10.2 | 5.3 | 52.4 | 38.2 | 9.1 |
| 012 | 5 | 1.4 | 5.0 | 27 | 270 | 19.3 | 13.2 | 68.7 | 21.4 | 9.9 |
| 013 | 6 | 1.4 | 5.0 | 7 | 270 | 10.9 | 9.1 | 83.5 | 11.1 | 0.0 |
| 014 | 6 | 1.4 | 5.0 | 23 | 270 | 10.1 | 9.2 | 90.5 | 9.8 | 0.0 |
| 015 | 6 | 1.4 | 5.0 | 75 | 270 | 10.4 | 8.8 | 85.1 | 6.5 | 0.0 |
| 016 | 6R | 1.4 | 5.0 | 23 | 270 | 10.0 | 8.9 | 88.7 | 11.1 | 0.0 |
| R = Regnerierter Katalysator von Beispiel 15 | | | | | | | | | | |
| 017 | 12 | 1.4 | 5.0 | 2 | 270 | 13.4 | 7.1 | 53.0 | 29.7 | 13.1 |
| 018 | 13 | 1.4 | 5.0 | 2 | 270 | 9.5 | 4.4 | 46.3 | 13.6 | 15.6 |
| 019 | 8 | 1.4 | 5.0 | 6 | 270 | 2.9 | 0.0 | 0.0 | 0.0 | 0.3 |
| 020 | 9 | 1.4 | 5.0 | 7 | 270 | 0.9 | 0.0 | 0.0 | 0.0 | 0.0 |

Vergleichsbeispiele 21 bis 22

4-Hydroxybenzaldehyd aus 4-Methoxybenzaldehyd ohne Wasserzusatz

Ausgangsstoff B: Lösung von 1 Mol 4-Methoxybenzaldehyd (II) und 3 Molen Methanol

Die Umsetzungen werden auf gleiche Weise wie im Beispiel 1 durchgeführt. Jedoch wird ohne Wasserzusatz gearbeitet. Die Ergebnisse sind in der Tabelle 2 aufgeführt.

Tabelle 2

| Vergleichsbeispiele ohne Wasserzusatz | | | | | | | | | | |
|---|---|---|---|---|---|---|---|---|---|---|
| Beispiel | Katalysator | WHSV/h | $N_2$ [Nl/h] | Laufzeit [h] | Temp. [°C] | GC-Analyse des Austrags | | | | |
| | | | | | | Umsatz an (II) [%] | Menge an (I) [Fl.-%] | Selekt. in [%] | | |
| | | | | | | | | (I) | (III) | (IV) |
| 021 | 4 | 1.4 | 5.0 | 6 | 350 | 28.5 | 5.5 | 19.3 | 51.7 | 13.3 |
| 022 | 4 | 1.4 | 5.0 | 6 | 270 | 2.1 | 1.5 | 72.5 | 27.5 | 0.0 |

Beispiele 23 bis 42

4-Hydroxybenzaldehyd aus 4-Methoxybenzaldehyd

Ausgangsstoff C: Emulsion aus 4-Methoxybenzaldehyd und Wasser im Molverhältnis von 1 : 1

Die Umsetzungen werden auf ähnliche Weise wie in den Beispielen 1 bis 19 durchgeführt. Jedoch wird mit Wasserzusatz aber ohne Methanolzusatz gearbeitet. Dabei werden Ausgangsstoff und Reaktionsprodukt, und der Stickstoff-Inertisierungsstrom im Gleichstrom, in absteigender Fahrweise über das Katalysatorfestbett geleitet. Mit Hilfe dieser absteigenden Fahrweise ist auch eine Umsetzung flüssiger Phasen möglich. Beispielsweise wird bei 200°C das Wasser in Form von Wasserdampf, p-Methoxybenzaldehyd (Siedepunkt 248°C) jedoch noch weitgehend als Flüssigkeit vorliegen. Auch in diesem Fall wird der gewünschte p-Hydroxybenzaldehyd erhalten.

Tabelle 3

| Beispiel | Katalysator | WHSV/h | $N_2$ [Nl/h] | Laufzeit [h] | Temp. [°C] | GC-Analyse des Austrags | | | | |
|---|---|---|---|---|---|---|---|---|---|---|
| | | | | | | Umsatz an (II) [%] | Menge an (I) [Fl.-%] | Selekt. in [%] | | |
| | | | | | | | | (I) | (III) | (IV) |
| 23 | 4 | 1.4 | 5.0 | 15 | 250 | 23.4 | 15.6 | 61.5 | 22.3 | 2.6 |
| 24 | 4 | 2.8 | 5.0 | 15 | 250 | 21.2 | 11.5 | 54.2 | 11.5 | 7.0 |
| 25 | 4 | 1.4 | 5.0 | 4 | 350 | 43.6 | 12.5 | 28.6 | 36.1 | 25.3 |
| 26 | 5 | 1.4 | 5.0 | 15 | 250 | 22.6 | 12.3 | 54.5 | 14.6 | 8.3 |
| 27 | 3 | 1.4 | 5.0 | 15 | 250 | 33.4 | 19.0 | 57.0 | 23.6 | 18.4 |
| 28 | 10 | 1.4 | 5.0 | 15 | 250 | 28.5 | 14.0 | 49.0 | 18.7 | 12.3 |
| 29 | 10R | 1.4 | 5.0 | 15 | 250 | 24.4 | 14.0 | 57.4 | 21.6 | 20.9 |
| R = regnerierter Katalysator von Beispiel 28 | | | | | | | | | | |
| 30 | 10 | 10.0 | 5.0 | 15 | 250 | 23.2 | 8.9 | 38.3 | 35.7 | 26.0 |
| 31 | 1 | 1.4 | 5.0 | 7 | 250 | 40.5 | 19.8 | 48.9 | 20.0 | 15.6 |
| 32 | 6 | 1.4 | 5.0 | 25 | 250 | 14.1 | 13.5 | 95.5 | 4.5 | 0.0 |
| 33 | 6 | 1.4 | 5.0 | 7 | 250 | 10.0 | 10.0 | 100 | 0.0 | 0.0 |
| 34 | 6 | 1.4 | 5.0 | 31 | 250 | 28.6 | 25.7 | 89.8 | 4.4 | 0.0 |
| Produkt von Beispiel 32 noch 2x über das Katalysatorbett geführt | | | | | | | | | | |
| 35 | 10 | 1.4 | 5.0 | 7 | 250 | 12.2 | 8.9 | 73.2 | 12.0 | 8.1 |
| 36 | 2 | 1.4 | 5.0 | 7 | 350 | 32.6 | 6.7 | 20.6 | 46.6 | 20.6 |
| 37 | 14 | 1.4 | 5.0 | 2 | 250 | 11.2 | 10.4 | 92.6 | 7.5 | 0.0 |
| 38 | 15 | 1.4 | 5.0 | 4 | 250 | 7.0 | 6.2 | 88.2 | 6.8 | 5.0 |
| 39 | 16 | 1.4 | 5.0 | 4 | 250 | 9.7 | 9.0 | 93.2 | 6.9 | 0.0 |
| 40 | 17 | 1.4 | 5.0 | 7 | 250 | 13.8 | 9.6 | 69.5 | 17.9 | 9.4 |
| 41 | 8 | 1.4 | 5.0 | 7 | 250 | 1.4 | 0.7 | 51.8 | 0.0 | 0.0 |
| 42 | 9 | 1.4 | 5.0 | 7 | 250 | 0.0 | 0.0 | 00.0 | 0.0 | 0.0 |

Beispiel 43

4-Hydroxybenzaldehyd (I) aus 4-t.-Butyloxybenzaldehyd (II)

Ausgangsstoff D: Emulsion aus 4-tertiär-Butyloxybenzaldehyd und Wasser im Molverhältnis von 1 : 1

Die Umsetzung werden auf ähnliche Weise wie im Beispiel 23 in der absteigenden Fahrweise durchgeführt.

Tabelle 4

| Beispiel | Katalysator | WHSV/h | $N_2$ [Nl/h] | Laufzeit [h] | Temp. [°C] | GC-Analyse des Austrags | | | |
|---|---|---|---|---|---|---|---|---|---|
| | | | | | | Umsatz an (II) [%] | Menge an (I) [Fl.-%] | Selekt. in [%] | |
| | | | | | | | | (I) | (III) |
| 43 | 6 | 1.4 | 5.0 | 2 | 150 | 100.0 | 100.0 | 100 | 00.0 |

Beispiel 44

4-Hydroxybenzaldehyd (I) aus 4-Benzyloxybenzaldehyd (II)

Ausgangsstoff E: Lösung aus 4-Benzyloxybenzaldehyd, Methanol und Wasser im Molverhältnis 0.1 : 12 : 2.5

Die Umsetzungen werden auf ähnliche Weise wie im Beispiel 23 in der absteigenden Fahrweise durchgeführt.

Tabelle 5

| Beispiel | Katalysator | WHSV/h | $N_2$ [Nl/h] | Laufzeit [h] | Temp. [°C] | GC-Analyse des Austrags | | | |
|---|---|---|---|---|---|---|---|---|---|
| | | | | | | Umsatz an (II) [%] | Menge an (I) [Fl.-%] | Selekt. in [%] | |
| | | | | | | | | (I) | (III) |
| 44 | 3 | 1.4 | 5.0 | 6 | 250 | 100.0 | 22.6 | 22.6 | 27.7 |

Beispiele 45 bis 49

Flüssigphasenreaktionen unter Druck

Ausgangsstoff A: Emulsion aus 4-Methoxybenzaldehyd : Methanol : Wasser
Molverhältnis: 1 : 1.5 : 1.5

Ausgangsstoff C: Emulsions 4-Methoxybenzaldehyd und Wasser im Molverhältnis 1:1

Die Ausgangsstoffe werden mit einer handelsüblichen Hochdruck-Dosierpumpe in den modifizierten Rohrreaktor eingetragen. Über Druckhalteventile auf der Produktaustragsseite wird der Druck im Rohrreaktor geregelt. Um eine gleichmäßige Fahrweise zu gewährleisten liegt der eintragsseitige Arbeitsdruck der Pumpe stets 1 - 2 bar über dem des austragseitigen Druckregelsystems, der beispielsweise auf 15 bis 20 bar eingestellt wird.

Tabelle 6

| Beispiel | Katalysator | WHSV/h | Ausgangsstoff | Laufzeit [h] | Temp. [°C] | GC-Analyse des Austrags | | | | |
|---|---|---|---|---|---|---|---|---|---|---|
| | | | | | | Umsatz an (II) [%] | Menge an (I) [Fl.-%] | Selekt. in [%] | | |
| | | | | | | | | (I) | (III) | (IV) |
| 045 | 6 | 1.4 | A | 7 | 250 | 11.3 | 10.7 | 94.3 | 5.6 | 0.0 |
| 046 | 1 | 1.4 | C | 4 | 240 | 28.6 | 20.3 | 70.9 | 15.8 | 13.2 |
| 047 | 6 | 1.4 | C | 6 | 240 | 14.4 | 13.1 | 90.8 | 3.9 | 3.1 |
| 048 | 3 | 1.4 | C | 10 | 240 | 31.4 | 21.7 | 69.2 | 14.0 | 14.3 |
| 049 | 4 | 1.4 | C | 6 | 240 | 24.2 | 13.2 | 54.4 | 3.9 | 4.7 |

Beispiele 50 bis 57

2-Hydroxybenzaldehyd (I) aus 2-Methoxybenzaldehyd (II)

Ausgangsstoff G: Emulsion aus 2-Methoxybenzaldehyd und Wasser im Molverhältnis 1 : 2

Ausgangsstoff H: Gemisch aus 2-Methoxybenzaldehyd, Methanol und Wasser im Molverhältnis 1 : 1.5 : 1.5

Ausgangsstoff I: Emulsion aus 2-Methoxybenzaldehyddimethylacetal und Waser im Molverhältnis 1:2

Die Umsetzungen werden in Anlehnung an Beispiel 1 als Gasphasenreaktion durchgeführt.

Tabelle 7

| Beispiel | Katalysator | WHSV/h | Ausgangsstoff | Laufzeit [h] | Temp. [°C] | GC-Analyse des Austrags | | | | |
|---|---|---|---|---|---|---|---|---|---|---|
| | | | | | | Umsatz an (II) [%] | Menge an (I) [Fl.-%] | Selekt. in [%] | | |
| | | | | | | | | (I) | (III) | (IV) |
| 050 | 1 | 1.4 | I | 2 | 250 | 43.9 | 30.8 | 70.1 | 13.8 | 13.2 |
| 051 | 1 | 1.4 | G | 4 | 240 | 76.7 | 27.9 | 36.4 | 24.3 | 38.4 |
| 052 | 1 | 1.4 | G | 2 | 300 | 90.1 | 9.2 | 10.1 | 30.4 | 45.0 |
| 053 | 7 | 0.7 | G | 6 | 250 | 58.4 | 41.6 | 71.3 | 18.2 | 30.6 |
| 054 | 7 | 1.4 | G | 6 | 250 | 36.1 | 28.2 | 78.1 | 8.9 | 9.5 |
| 055 | 7 | 1.4 | H | 2 | 250 | 82.7 | 58.4 | 70.6 | 12.5 | 9.4 |
| 056 | 7 | 1.4 | H | 2 | 250 | 63.8 | 31.6 | 49.6 | 16.1 | 29.4 |
| | | | | 29 | 250 | 26.4 | 22.1 | 84.0 | 9.1 | 6.4 |
| 057 | 7 | 1.4 | I | 2 | 250 | 66.1 | 35.7 | 54.0 | 12.1 | 22.5 |

Beispiele 58 bis 63

2-Hydroxybenzaldehyd (I) aus 2-Methoxybenzaldehyd (II)

Ausgangsstoff H: Gemisch aus 2-Methoxybenzaldehyd, Methanol und Wasser im Molverhältnis 1 : 1.5 : 1.5

Ausgangsstoff K: Emulsion aus 2-Methoxybenzaldehyd und Wasser im Molverhältnis 1 : 1

Ausgangsstoff L: Gemisch aus 2-Methoxybenzaldehyddimethylacetal, Methanol und Wasser im Molverhältnis 1 : 1.5 : 1.5

Die Umsetzungen werden in Anlehnung an Beispiel 45 als Flüssigphasenreaktion unter erhöhtem Druck bei 16 bis 18 bar durchgeführt.

Tabelle 8

| Beispiel | Katalysator | WHSV/h | Ausgangsstoff | Laufzeit [h] | Temp. [°C] | GC-Analyse des Austrags | | | | |
|---|---|---|---|---|---|---|---|---|---|---|
| | | | | | | Umsatz an (II) [%] | Menge an (I) [Fl.-%] | Selekt. in [%] | | |
| | | | | | | | | (I) | (III) | (IV) |
| 058 | 1 | 1.4 | H | 1 | 240 | 8.6 | 5.9 | 68.9 | 14.4 | 0.0 |
| 059 | 1 | 1.4 | H | 6 | 240 | 16.3 | 14.1 | 86.2 | 9.2 | 0.0 |
| 060 | 1 | 1.4 | K | 1 | 240 | 53.3 | 32.9 | 61.6 | 29.5 | 4.7 |
| 061 | 1 | 1.4 | K | 6 | 240 | 11.5 | 9.7 | 84.4 | 9.1 | 0.0 |
| 062 | 1 | 1.4 | L | 1 | 240 | 55.9 | 39.9 | 71.4 | 18.2 | 0.0 |
| 063 | 1 | 1.4 | L | 2 | 240 | 28.0 | 23.6 | 84.2 | 12.3 | 0.0 |

Beispiele 64 bis 65

3-Hydroxybenzaldehyd (I) aus 3-Methoxybenzaldehyd (II) Gasphasenreaktion entsprechend Beispiel 23.

Ausgangsstoff M: Emulsion aus 3-Methoxybenzaldehyd und Wasser im Molverhältnis 1 : 2

Tabelle 9

| Beispiel | Katalysator | WHSV/h | Ausgangsstoff | Laufzeit [h] | Temp. [°C] | GC-Analyse des Austrags | | | | |
|---|---|---|---|---|---|---|---|---|---|---|
| | | | | | | Umsatz an (II) [%] | Menge an (I) [Fl.-%] | Selekt. in [%] | | |
| | | | | | | | | (I) | (III) | (IV) |
| 064 | 1 | 1.4 | M | 4 | 250 | 56.0 | 54.9 | 98.1 | 0.0 | 0.0 |
| 065 | 7 | 1.4 | M | 4 | 250 | 55.9 | 54.3 | 97,1 | 0.0 | 0.0 |

**Patentansprüche**

**1.** Verfahren zur Herstellung von Hydroxybenzaldehyden der Formel (I) aus Benzaldehyden der Formel (II) oder deren Acetalen, in denen $R^1$ in der $R^1$O-Phenylethergruppe einen Alkyl-, Cycloalkyl-, Alkylcycloalkyl- oder einen Alkylarylrest bedeutet und $R^2$ = H, OH oder mit $R^1$ identisch ist, dadurch gekennzeichnet, daß man Benzaldehyde der Formel (II) oder deren Acetale, in denen $R^1$ und $R^2$ die vorgenannte Bedeutung aufweisen, in Anwesenheit von Wasser an Zeolithen als heterogene Katalysatoren zu Hydroxybenzaldehyden der Formel (I)

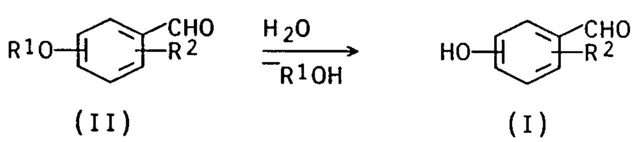


(II) (I)

umsetzt.

**2.** Verfahren zur Herstellung von Monohydroxybenzaldehyden nach Anspruch 1 aus Benzaldehyden oder deren Acetalen, in denen $R^1$ in der $R^1$O-Phenylethergruppe einen Alkyl- oder einen Alkylarylrest bedeutet und $R^2$ = H ist, dadurch gekennzeichnet, daß man Benzaldehyde der Formel (II) oder deren

Acetale, in denen $R^1$ und $R^2$ die vorgenannte Bedeutung aufweisen und die $R^1$O-Phenylethergruppe die vorgenannte Stellung in bezug auf die Aldehydfunktion einnimmt, in Anwesenheit von Wasser an Zeolithen als heterogene Katalysatoren zu Hydroxybenzaldehyden der Formel (I) umsetzt.

**3.** Verfahren nach Ansprüchen 1 und 2, dadurch gekennzeichnet, daß man als Katalysatoren Zeolithe des Pentasil-Typs verwendet.

**4.** Verfahren nach Ansprüchen 1 und 2, dadurch gekennzeichnet, daß man als Katalysatoren Aluminosilikatzeolithe verwendet.

**5.** Verfahren nach Ansprüchen 1 und 2, dadurch gekennzeichnet, daß man als Katalysatoren Borosilikatzeolithe verwendet.

**6.** Verfahren nach Ansprüchen 1 und 2, dadurch gekennzeichnet, daß man als Katalysatoren Eisensilikatzeolithe verwendet.

**7.** Verfahren nach Ansprüchen 3 bis 6, dadurch gekennzeichnet, daß man als Katalysatoren mit Alkali- und/oder Erdalkalimetall dotierte Zeolithe verwendet.

**8.** Verfahren nach Ansprüchen 3 bis 6, dadurch gekennzeichnet, daß man mit Übergangsmetallen dotierte Zeolithe als Katalysatoren verwendet.

**9.** Verfahren nach Ansprüchen 3 bis 6, dadurch gekennzeichnet, daß man mit seltenen Erdmetallen dotierte Zeolithe als Katalysatoren verwendet.

**10.** Verfahren nach Ansprüchen 3 bis 6, dadurch gekennzeichnet, daß man als Katalysatoren mit Säuren behandelte Zeolithe verwendet.

**11.** Verfahren nach Ansprüchen 3 bis 6, dadurch gekennzeichnet, daß man als Katalysatoren mit Phosphor dotierte Zeolithe verwendet.

**12.** Verfahren nach Ansprüchen 3 bis 9, dadurch gekennzeichnet, daß man als Katalysatoren dotierte Zeolithe verwendet, die zusätzlich Phosphor enthalten.

## Claims

**1.** A process for the preparation of a hydroxybenzaldehyde of the formula (I) from a benzaldehyde of the formula (II) or its acetal, in which $R^1$ in the $R^1$O-phenyl ether group is an alkyl, cycloalkyl, alkylcycloalkyl or alkylaryl radical and $R^2$ is H or OH or is identical to $R^1$, wherein a benzaldehyde of the formula (II) or its acetal, in which $R^1$ and $R^2$ have the abovementioned meanings, is converted into a hydroxybenzaldehyde of the formula (I)

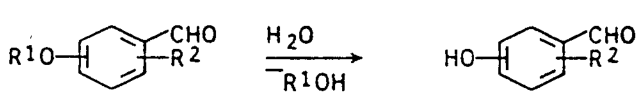


(II) (I)

in the presence of water over a zeolite as a heterogeneous catalyst.

**2.** A process for the preparation of a monohydroxybenzaldehyde as claimed in claim 1 from a benzaldehyde or its acetal in which $R^1$ in the $R^1$O-phenyl ether group is an alkyl or alkylaryl radical and $R^2$ is H, wherein a benzaldehyde of the formula (II) or its acetal, in which $R^1$ and $R^2$ have the abovementioned meanings and the $R^1$O-phenyl ether group occupies the abovementioned position with respect to the aldehyde function, is converted into a hydroxybenzaldehyde of the formula (I) in the presence of water over a zeolite as a heterogeneous catalyst.

3. A process as claimed in claims 1 and 2, wherein the catalyst used is a zeolite of the pentasil type.

4. A process as claimed in claims 1 and 2, wherein the catalyst used is an aluminosilicate zeolite.

5. A process as claimed in claims 1 and 2, wherein the catalyst used is a borosilicate zeolite.

6. A process as claimed in claims 1 and 2, wherein the catalyst used is a ferrosilicate zeolite.

7. A process as claimed in claim 3 or 4 or 5 or 6, wherein the catalyst used is a zeolite doped with an alkali metal and/or an alkaline earth metal.

8. A process as claimed in claim 3 or 4 or 5 or 6, wherein the catalyst used is a zeolite doped with transition metals.

9. A process as claimed in claim 3 or 4 or 5 or 6, wherein the catalyst used is a zeolite doped with rare earth metals.

10. A process as claimed in claim 3 or 4 or 5 or 6, wherein the catalyst used is a zeolite treated with acids.

11. A process as claimed in claim 3 or 4 or 5 or 6, wherein the catalyst used is a zeolite doped with phosphorus.

12. A process as claimed in claim 3 or 4 or 5 or 6 or 7 or 8 or 9, wherein the catalyst used is a doped zeolite which additionally contains phosphorus.

**Revendications**

1. Procédé de préparation d'hydroxybenzaldéhydes de formule (I) à partir de benzaldéhydes de formule (II) ou d'acétals de ceux-ci, dans lesquels $R^1$ dans le groupement $R^1$O-oxyphényle représente un reste alkyle, cycloalkyle, alkylcycloalkyle ou alkylaryle et $R^2$ = H, OH ou est identique à $R^1$, caractérisé en ce qu'on transforme en hydroxybenzaldéhydes de formule (I) des benzaldéhydes de formule (II) ou des acétals de ceux-ci, dans lesquels $R^1$ et $R^2$ ont les significations sus-indiquées, en présence d'eau et de zéolithes servant de catalyseurs hétérogènes

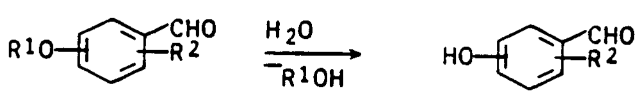


(II) (I)

2. Procédé de préparation de monohydroxybenzaldéhydes selon la revendication 1 à partir de benzaldéhydes ou d'acétals de ceux-ci, dans lesquels $R^1$ dans le groupement $R^1$O-oxyphényle représente un reste alkyle ou alkylaryle et $R^2$ = H, caractérisé en ce qu'on transforme en hydroxybenzaldéhydes de formule (I) des benzaldéhydes de formule (II) ou des acétals de ceux-ci, dans lesquels $R^1$ et $R^2$ ont les significations sus-indiquées et le groupement $R^1$O-oxyphényle occupe la position sus-indiquée par rapport à la fonction aldéhyde, en présence d'eau et de zéolithes servant de catalyseurs hétérogènes.

3. Procédé selon la revendication 1 ou 2, caractérisé en ce qu'on utilise, comme catalyseurs, des zéolithes du type pentasil.

4. Procédé selon la revendication 1 ou 2, caractérisé en ce qu'on utilise, comme catalyseurs, des zéolithes d'aluminosilicate.

5. Procédé selon la revendication 1 ou 2, caractérisé en ce qu'on utilise, comme catalyseurs, des zéolithes de borosilicate.

**6.** Procédé selon la revendication 1 ou 2, caractérisé en ce qu'on utilise, comme catalyseurs, des zéolithes de ferrosilicate.

**7.** Procédé selon l'une quelconque des revendications 3 à 6, caractérisé en ce qu'on utilise, comme catalyseurs, des zéolithes dopées avec des métaux alcalins et/ou alcalino-terreux.

**8.** Procédé selon l'une quelconque des revendications 3 à 6, caractérisé en ce qu'on utilise, comme catalyseurs, des zéolithes dopées avec des métaux de transition.

**9.** Procédé selon l'une quelconque des revendications 3 à 6, caractérisé en ce qu'on utilise, comme catalyseurs, des zéolithes dopées avec des métaux des terres rares.

**10.** Procédé selon l'une quelconque des revendications 3 à 6, caractérisé en ce qu'on utilise, comme catalyseurs, des zéolithes traitées par des acides.

**11.** Procédé selon l'une quelconque des revendications 3 à 6, caractérisé en ce qu'on utilise, comme catalyseurs, des zéolithes dopées avec du phosphore.

**12.** Procédé selon l'une quelconque des revendications 3 à 9, caractérisé en ce qu'on utilise, comme catalyseurs, des zéolithes dopées qui contiennent en plus du phosphore.